# EUROPEAN PATENT SPECIFICATION

(11) **EP 2 600 915 B1**
(45) Date of publication and mention of the grant of the patent: **04.03.2015**
(21) Application number: 11743426.6
(22) Date of filing: 05.08.2011
(51) Int. Cl.: A61L 29/14, A61M 25/00

(54) **FLEXIBLE WATER VAPOUR BARRIER TUBE FOR PACKAGING PURPOSE**
WASSERDAMPFSPERRSCHLAUCH FÜR VERPACKUNGSZWECKE
TUBE FLEXIBLE ÉTANCHE À LA VAPEUR D'EAU UTILISABLE EN TANT QU'EMBALLAGE

(30) Priority: 05.08.2010 DK 201070351
(43) Date of publication of application: 12.06.2013
(73) Proprietor: Coloplast A/S, 3050 Humlebæk (DK)
(72) Inventor: NIELSEN, Henrik Lindenskov, DK-2765 Smoerum (DK)
(86) International application number: PCT/DK2011/050305
(87) International publication number: WO 2012/016571

(56) References cited:
- EP-A1- 0 679 506
- WO-A1-97/47349
- WO-A1-98/19729
- WO-A1-99/30761
- CA-A1- 2 116 009
- GB-A- 2 319 507

## Description

### Field of the invention

The present invention relates to a tubular package for a urinary catheter. Furthermore, the invention relates to a catheter package set comprising a tubular package, a catheter and a swelling medium.

### Background

Urinary catheters are used as a tool for assisting in the draining of the urinary bladder of persons that have reduced or non-existing bladder control. The reduced or non-existing bladder control may either be temporary or permanent, where a temporary loss of bladder control may be caused by trauma, loss of consciousness or illness, as an example. An example of a permanent loss of bladder control may be, where a loss of a neural connection between the brain or spinal cord and the urinary bladder occurs due to a trauma to the spinal cord, as is often the case with para- and tetraplegics.

One example of a urinary catheter which is widely used for draining urine from the urinary bladder, is where a catheter tube is inserted into the urethra of a user and where the tip of the catheter tube is manoeuvred into the urinary bladder, forcing the urethral sphincter open and thus providing a drainage channel from the urinary bladder and out of the body, via the catheter tube. There are two types of catheters which are commonly used, the permanent catheter and the intermittent catheter. The permanent catheter is a highly flexible catheter which is inserted by medical professionals into the body for a long period of time and where the catheter is anchored inside the bladder. The intermittent catheter is usually a single use catheter or a multiple use catheter, which is inserted by the user into the urethra/bladder for the immediate drainage of their urinary bladder and is removed from the urethra/bladder subsequent to the drainage.

There are a number of different types of intermittent catheters which are currently available for the user, such as the SpeediCath® and EasiCath® marketed by Coloplast A/S. These catheters are conventional one-piece catheter tubes which have an outlet at their distal end which may be used to connect the catheters to a urinary bag for collecting the urine drained from the urine bladder.

Catheters are usually coated with a hydrophilic coating. The coating is as a minimum applied on that part of the surface which is introduced or comes into contact with e.g. mucous membranes during introduction of the device. Whereas such a coating is not particularly slippery when dry, it may become extremely slippery when it is swelled with water before introduction into the human body. The hydrophilic coating thus ensures a substantially painless introduction with a minimum of damage on tissue.

Ready to use hydrophilic coated intermittent catheters are usually packed with a swelling medium, such as water, and optionally containing various additives.

Some catheter packaging concepts are based on tubes instead of packaging films or foils.

The problem has until now been to secure that such a tube is flexible and at the same time gives satisfactory shelf life for the product.

Conveen® Expect catheter, the first ever ready to use hydrophilic coated intermittent catheter, was launched in year 2000 by Coloplast A/S. This product was packed in corrugated tubing made of 14% polyethylene vinyl acetate copolymer (EVA) material. The tube had good flexibility but had poor water barrier properties. Therefore, it was delivered with an aluminium bag as a secondary packaging.

SpeediCath® Compact Female catheter was launched in 2004. This product was primarily packed in a 1 mm thick polypropylene (PP) homopolymer tubing which gives the required water barrier. However, this tube has no flexibility.

EP 2,106,821 discloses a catheter assembly comprising a catheter having an insertion end and a discharge end, and a receptacle. The receptacle comprises a tubular member which in a storage state accommodates a catheter section including the insertion end of the catheter. The tubular member has at least one pleated region located along the tubular member arranged with a plurality of pleats, which pleats in the storage state forms a curvature, thereby enabling for a curved disposition of a catheter section located therein. The catheter assembly allows the catheter, during storage, to be curved such that minimum space consumption is required, meanwhile kinking of the catheter is avoided.

WO 2007/050685 discloses a pre-wetted intermittent catheter apparatus including a collapsible container. The catheter apparatus includes a container made of a gas impermeable material such as polypropylene and polyethylene. The pleat or folds in the outer surface of the container permit the container to compress or collapse.

CA2116009 A discloses a sealed elongated envelope encapsulating a catheter, said envelope being constructed of flexible plastic sheet material.

From the above examples it can be seen that there is a need at the same time to fulfil the water barrier and the flexibility properties, when packaging ready to use hydrophilic coated catheters in a tube package.

### Summary of the invention

The present invention provides a flexible tubular package. The package is for an intermittent catheter. The intermittent catheter is coated with a hydrophilic coating, which becomes slippery when in contact with a swelling medium. Thus, the tubular package of the invention can withhold the swelling medium for a long time and at the same time being flexible and bendable.

Furthermore, the present invention provides a catheter package set, which comprises a tubular package, an intermittent catheter and a swelling medium.

### Brief description of the drawings

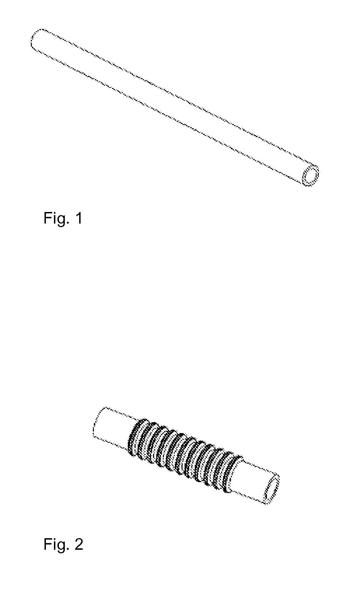

Figure 1 illustrates one embodiment of the invention, wherein the tube is straight.
Figure 2 illustrates an embodiment of the invention, wherein the tube is corrugated.

### Detailed description of the present invention

**Abbreviations and Classification of materials:**

| Trade name/ trivial name/ abbreviation | Chemical name |
|---|---|
| **Thermoplastic polymers:** | |
| ***Polyolefin based:*** | |
| PE | Polyethylene (either homopolymer or copolymer) |
| LDPE | Low density polyethylene (homopolymer) |
| LLDPE | Linear low density polyethylene (including copolymers with butene, hexene and octene) |
| HDPE | High Density Polyethylene |
| PP | Polypropylene, homopolymer |
| PP-cop. | Polypropylene copolymers including e.g. Vistamaxx from ExxonMobile |
| EVA | Poly-ethylene-vinyl-acetate copolymer |
| EMA | Poly-ethylene-methyl-acrylate copolymer |
| EEA | Poly-ethylene-ethyl-acrylate copolymer |
| EBA | Poly-ethylene-butyl-acrylate copolymer |
| COC | Cyclic polyolefin, e.g. Topas from Ticona |
| COC-PE | Compound of cyclic polyolefin and polyethylene |

| ***Halogen based:*** | |
|---|---|
| PVC | Polyvinyl Chloride |
| PVDC | Polyvinylidene Chloride, e.g. Selar from Dow Chemical |
| PCTFE | Polychlortriflourethylene e.g. Aclar from Honeywell |

| **Thermoplastic Elastomers:** | |
|---|---|
| ***Styrene based:*** | |
| SiBS | Polystyrene-*block*-poly(isobutylene)-*block*-polystyrene, e.g. Sibstar from Kaneka |
| SEBS | Polystyrene-*block*-poly(ethylene/butylene)-*block*-polystyrene, e.g. Kraton G from Kraton |
| SEPS | Polystyrene-*block*-poly(ethylene/propylene)-*block*-polystyrene, e.g. Septon from Kuraray |
| SBS | Polystyrene-*block*-poly(butadiene)-*block*-polystyrene, e.g. Kraton D (SBS types) |
| SIBS | Polystyrene-*block*-poly(Isoprene/butadiene)-*block*-polystyrene, e.g. Kraton D (SIBS types) |
| SIS | Polystyrene-*block*-poly(Isoprene)-*block*-polystyrene, e.g. Kraton D (SIS types) |

| ***Other polyolefin:*** | |
|---|---|
| PIB | Polyisobutylene |
| IIR | Uncured butyl rubber (poly-isobutylene-isoprene copolymer, e.g. Lanxess Butyl) |
| PP/EPDM | Polypropylene with in-situ crosslinked ethylene-propylene-diene-methylene rubber e.g. Santoprene from ExxonMobile Chemical |

| ***Halogen based:*** | |
|---|---|
| CIIR | Chlorobutyl uncured rubber |
| BIIR | Bromobutyl uncured rubber |

The aim of the invention is to secure that a tubular package for an intermittent catheter is flexible and bendable and at the same time gives satisfactory shelf life for the product. The goal is achieved by the choice of the tube material.

A good shelf life of the products is achieved by keeping the catheter wet by the swelling medium at all times. Thus, the package should avoid leakage of any kind of the swelling medium from the package.

In an embodiment of the invention, a catheter package is formed as a tube for an intermittent catheter, wherein the tube material is flexible and has a low water vapour transmission rate.

By tube or tubular is meant a cylinder-like element with a substantially circular cross section.

The tube material has a water vapour transmission rate below 0.50 g/m²/mm/24h, preferably below 0.30 g/m²/mm/24h.

Water vapour transmission rate of the tube material can be measured according to standard method ASTM E96.

The catheter package of the invention is based on tubes instead of packaging films or foils. This gives advantages in making products which are very compact and discrete. The tubular product is easy to have in the pocket or bag. Moreover, the product is easier to handle and is more visually appealing.

Furthermore, a tubular package may give other product features and benefits such as using the tubular package as an extender tube to the toilet. For this intended use, it is also important that the tube has a certain flexibility and kink resistance.

It is also a requirement that the package can be radiation sterilized without degradation of the material and with only very small change of the geometrical dimensions.

As the standard solution today is to use a packaging based on aluminium foil, the package of the invention could also give an environmental advantage.

The packages disclosed in the prior art do not fulfill both the requirements with regards to barrier properties and to flexibility properties. Either the packages were using hard water barrier polymers like PP and HDPE in a thickness of 0.5 to 1.0 mm or they were soft as e.g. EVA and needed an extra secondary packaging to obtain the water vapour barrier requirements.

The barrier properties alone can be fulfilled by standard materials such as PE or PP or more special materials COC, PVDC or Fluor polymers, however these are not very flexible and some of them have high costs and a poor environmental profile.

The flexibility properties can be fulfilled by using PE copolymers like EVA, EMA, EEA, EBA, or metallocene PP copolymers (like Vistamaxx; Exxon Chemical and, Versify; Dow Chemical) or by thermoplastic elastomer materials like SBS, SEBS, SEPS compounds or EPDM/PP compounds. Some of these have water vapour barrier properties to some extent. However, for packaging of a hydrophilic coated catheter, they have not at the same time been fulfilling the requirements for shelf life, flexibility, kink resistance, sterilization stability, environment and cost price.

Standard polyolefins like PE or PP have rather good water vapour barrier properties. However to get the water vapour barrier properties, it must be very unpolar (having essentially no polar co-monomers), and be very high crystalline. This together with the required wall thickness makes the package very inflexible.

For these unpolar polyolefins the barrier properties are well correlated with the percentage of crystalinity. For PP the crystalinity is mainly dependent of the ethylene co-monomer content and for some block copolymer types also on the polymerization process. For PE polymers the crystalinity is mainly depending on the branching defined polymerization process and on the content of unpolar co-monomers like butylenes, hexene and octene. PE is normally divided into 3 main categories, HDPE, LDPE and LLDPE. HDPE is quite linear and LDPE are more branched in structure. LLDPE is very linear in overall structure, but has the small scale branching defined by the co-monomers. For the same density LDPE and LLDPE are very similar in water vapour barrier and in flexibility. However, LLDPE has a higher melting point and thus better high temperature performance, which may be important in the sterilization process.

According to an embodiment of the invention, the composition of the tube material comprises polyethylene which is a low density polyethylene or a linear low density polyethylene.

By low density polyethylene (LDPE) is meant a thermoplastic polymer (polyethylene), wherein the LDPE is defined by a density range of 0.910 - 0.940 g/cm³.

By linear low density polyethylene (LLDPE) is meant a substantially linear polymer (polyethylene), with significant numbers of short branches, commonly made by copolymerization of ethylene with longer-chain olefins. Linear low-density polyethylene differs structurally from conventional low-density polyethylene because of the absence of long chain branching.

Standard PE's and PP's are too stiff for a straight flexible tubing. However, some specialty grades of metallocene LLDPE/VLDPE and PP-PE copolymers with modulus below 100 MPa can be used. Examples of these materials are Clearflex (LLDPE from Polymeri) with densities below 0.91 g/cm³ and Vistamaxx and Versify (metallocene PP/PE copolymers from Exxon and Dow Chemical). For corrugated tubing good flexibility can be obtained for standard PE's with E-modulus below 200MPa. However, the barrier will be reduced because of a higher surface area of a corrugated tube (typically double area) and thinner material (typically half thickness).

In one embodiment of the invention, the composition of the tube material comprises polyethylene with a density of 0.900-0.920 g/cm³.

A LLDPE with a density between 0,900 g/cm³ and 0,920 g/cm³ is a suitable compromise between flexibility, water vapour barrier and dimensional stability at radiation sterilization temperatures.

According to one embodiment of the invention, the composition of the tube material comprises a thermoplastic polymer.

According to an embodiment of the invention, the composition of the tube material comprises a thermoplastic elastomer.

Thermoplastic elastomer compounds can be very soft and unpolar compounds. Such compounds may be based on SEBS together with PP or PE and in many cases with paraffin oil as a plasticizer. A customized compound can give the required flexibility, kink-resistance and water vapour barrier for a straight tube, and in the best cases it can also fulfil the water vapour barrier requirements for a corrugated tube.

According to an embodiment of the invention, the thermoplastic elastomer further comprises polypropylene and/or polyethylene, and optionally a plasticizer such as paraffin oil in a compound.

In one embodiment of the invention, the composition of the tube material contains 1-10% (w/w) of low molecular PE wax (Mw 500 to 1500 g/mol) in order to improve the barrier properties and flexibility.

By polyethylene wax (PE wax) is meant a low molecular weight polyethylene polymer that, because of its low molecular weight, has wax like physical characteristics.

Polyisobutylene is a polyolefin, which at the same time is soft and has very good water barrier properties, even much better than could be expected from its unpolar nature and low crystallinity. It could be explained by a molecular structure, which gives very low mobility of the polymer chains in the amorphous phase, even though the actual temperature is above the Tg for the amorphous phase of the material.

In its pure form, polyisobutylene is not a solid material, thus it needs to be used either as:
- Cured or uncured butyl rubber (poly-isobutylene-isoprene copolymer e.g. Exxon Butyl, Lanxess Butyl).
- Block copolymer with e.g. polystyrene as the end blocks, SiBS, styrene-isobutylene-styrene (e.g. Sibstar from Kaneka).
- Compound with a thermoplastic material like polyethylene or polypropylene or styrene block-copolymers (e.g. SEBS, SEPS or SBS). These compound may contain polybutene, polyisobutylene, cured or uncured butyl rubber, and/or SiBS blockcopolymer.

According to one embodiment of the invention, the composition of the tube material comprises polybutene, polyisobutylene, cured or uncured butyl rubber, or block copolymer with polystyrene as the end blocks such as styrene-isobutylene-styrene.

According to another embodiment of the invention, the thermoplastic elastomer comprises a styrene block copolymer such as polystyrene-*block*-poly(ethylene/butylene)-*block-*polystyrene, polystyrene-*block*-poly(ethylene/propylene)-*block*-polystyrene, polystyrene-*block*-polybutadiene-*block*-polystyrene or polystyrene-*block*-polyisobutylene-*block-*polystyrene.

According to an embodiment of the invention, the tube is straight.

By a straight tube is meant a tube without pleats.

According to another embodiment of the invention, the tube is corrugated.

By a corrugated tube is meant a tube with a series of pleats or parallel ridges and furrows.

In one embodiment of the invention, the tube is a mono layer construction.

By a mono layer construction is meant a construction with one layer. The material of the layer consists of one composition.

The tube package is normally made in slightly more than the full length of the catheter, but can, for certain applications, be made in lengths up to e.g. 2 times, or even more, of the length of the catheter.

The tube can be closed in both ends with a hot melt plug. The tubes can also be closed by other secure means e.g. by welded or glued small caps or lids or simply closing the ends by welding the tube together.

In an embodiment of the invention, a catheter package set comprises a tube package, an intermittent catheter, a swelling medium and optionally a urine collection bag, wherein the tube material is flexible and has a low water vapour transmission rate.

In one embodiment of the invention, the swelling medium is disposed within the tube package.

According to an embodiment of the invention, the catheter package set comprises a tube package as described above.

According to one embodiment of the invention, the catheter package set comprises an intermittent catheter comprising a hydrophilic coating.

In one embodiment of the invention, the package set has been sterilized using irradiation while in contact with the swelling medium.

In a preferred embodiment of the invention, the set is sterilized using β- or y-irradiation.

### Experimental

### Materials tested

| | |
|---|---|
| LDPE grades tested were: | Riblene LH10 from Polimeri Europe |
| LLDPE grades were: | Flexirene CL10, Clearflex CLBO and Clearflex MPD0 |
| SEBS compound were: | Meliflex M6806 and R2783 from Melitek |
| SEBS compound with PE-wax were: | Meliflex R2677B-WX5 from Melitek |
| SIBS compounds tested: were: | Wittenburg Code B and Wittenburg Code C |
| SIBS were: | Sibstar T102 from Kaneka |

### Methods

Water permeability: The Water Vapour Transmission Rate (WVTR) at relevant environmental conditions. Standard measuring conditions often used in the literature 38°C with 90%RH difference, however 30°C and 40°C are also commonly used. The conditions used for the examples here are 30°C with 70% Relative Humidity difference (100%RH inside and 30%RH outside). For flat samples the permeability is linear correlated with the surface area and with the reciprocal thickness of the sample. For tube samples, a good and simple approximation has been made for the calculation as follows. For straight as well as corrugated tubing the values have been calculated by using the mean of the inside and the outside surface area and the mean material thickness. The calculations have been made on the results of the Water loss measurements, which have been performed on actual tube samples. See below.

Water loss: The water loss by permeation through the packaging is a combination of the actual geometrical design, the materials and the environmental conditions. The permitted water loss depends on the product design. For the water loss measurements the actual tubes in a length of 350 mm has been filled with 6 ml of the actual catheter swelling media (93% water 6% PVP polymer and 0,9% NaCl) and, for the testing, closed in both ends with a hot melt plug of a minimum of 4 mm in thickness. By weighing the samples a minimum of 3 times within a few months of storage at 30°C and 70%RH difference, a very good linear correlation can be made between the water loss and the storage time.

Flexibility: Subjective ability to be bent. For practical purposes the bending characteristics are correlated with the E modulus of the material and the geometrical size and shape. For relevant packaging tube sizes with an inside diameter of 6 to 10 mm and a wall thickness between 0.5 and 1.0 mm E-modulus up to 150 MPa can be used for straight tubing and up to 400 MPa for a tube with a corrugated design.
Sterilization: The catheter product is to be E-beam or Gamma sterilized.

### Water loss measurement

350 mm length of the tubes were closed in one end with polyethylene hotmelt and packed with 6 ml of the PVP catheter swelling medium before closing the other end with the PE hotmelt. The products were weighed on a calibrated scale with an accuracy of +/-0.1 mg. Results reported for each experiment are average for 3 measurements, and they have been corrected for the weight loss for reference samples without the swelling medium.

### Example 1:

1-layer corrugated tube
Surface Area: 102,5cm²

### Water loss

| No. | Tube material | Material Density g/cm³ | Tube Weight | Average thickness | Weight loss, 5 days | Weight loss, 15 days | Weight loss, 33 days | Calculated Weight Loss 2 years (Linear regression | Permeability Coefficient 30°C with 70%RH difference |
|---|---|---|---|---|---|---|---|---|---|
| | | g/cm3 | g | mm | g | g | g | g | g/m²/mm/24h |
| 1 | Flexirene CL10 LLDPE | 0,918 | 6,67 | 0,69 | 0,018 | 0,045 | 0,103 | 3,0 | 0,20 |
| 2 | Clearflex CLB0 LLDPE | 0,911 | 6,42 | 0,68 | 0,022 | 0,059 | 0,139 | 4,1 | 0,27 |
| 3 | Clearflex MPD0 LLDPE | 0,900 | 6,46 | 0,67 | 0,018 | 0,082 | 0,190 | 5,6 | 0,37 |
| 4 | Meliflex M6608 SEBS-Comp. | 0,90 | 6,92 | 0,69 | 0,041 | 0,107 | 0,240 | 7,0 | 0,47 |
| 5 | Meliflex R2783 TPE-Comp. | 0,91 | 7,05 | 0,68 | 0,026 | 0,067 | 0,149 | 4,3 | 0,29 |
| 6 | Meliflex R2677B-WX5 PE wax-modified SEBS-Comp. | 0,90 | 6,48 | 0,69 | 0,033 | 0,081 | 0,167 | 4,7 | 0,31 |

### Example 2:

1-layer straight tube
Surface Area: 67cm²

### Water loss

| No. | Tube material | Material Density g/cm3 | Tube Weight | Average thickness | Weight loss, 5 days | Weight loss, 15 days | Weight loss, 33 days | Calculated Weight Loss 2 years (Linear regression | Permeability Coefficient |
|---|---|---|---|---|---|---|---|---|---|
| | | g/cm3 | g | mm | g | g | g | g | g/m2/mm/24 |
| 7 | Wittenburg B SIBS compound | 0,90 | 6,67 | 0,72 | 0,018 | 0,045 | 0,103 | 0,78 (1,6 as corrugated) | 0,12 |
| 8 | Wittenburg C SEBS compound | 0,90 | 6,42 | 0,79 | 0,022 | 0,059 | 0,139 | 1,9 (4,1 as corrugated) | 0,31 |

### Flexibility

| No. | Tube material | Tube type | Average thickness | Modulus ISO 527 | Flexibility | Kink | Length Stability in E-beam sterilization |
|---|---|---|---|---|---|---|---|
| | | | mm | MPa | Actual | Construction | |
| 1 | Flexirene CL10 LLDPE | Corrugated | 0,69 | 90 MPa | Acceptable | Acceptable | Neutral |
| 2 | Clearflex CLB0 LLDPE | Corrugated | 0,68 | 80 MPa | Acceptable | Acceptable | Neutral |
| 3 | Clearflex MPD0 LLDPE | Corrugated | 0,67 | 60 MPa | Acceptable | Acceptable | 2% Increase - not acceptable as corrugated |
| 4 | Meliflex M6608 SEBS-Comp. | Corrugated | 0,69 | 21,5 MPa | Very flexible | Acceptable | Not tested |
| 5 | Meliflex R2783 SEBS Comp. | Corrugated | 0,68 | Not tested | Very flexible | Acceptable | Not tested |
| 6 | Meliflex R2677B-WX5 PE wax-modified SEBS-Comp. | Corrugated | 0,69 | Not tested | Very flexible | Acceptable | Not tested |
| 7 | Wittenburg B SIBS compound | Straight | 0,72 | Not tested | Acceptable | Acceptable | Not tested |
| 8 | Wittenburg C SEBS compound | Straight | 0,79 | Not tested | Acceptable | Acceptable | Not tested |

### Discussion

The examples show that a satisfactory compromise can be obtained between the water loss, flexibility and sterilization properties for the tube either by using:
- A corrugated tube made in a polyethylene with a density of between 0,90 g/cm³ and 0,92 g/cm³.
- A corrugated tube or straight tube made in a TPE compound, for instance a SEBS compound.
- Especially well low water loss is obtained by using a TPE compound containing polyisobutylene, for instance in the form of SIBS (styrene-isobutylene-styrene)
- Also PE, PP homo and copolymers and PE-wax can be included as part of a TPE compound in order to get low water loss without compromising the flexibility.

## Claims

1. A catheter package formed as a tube for an intermittent catheter, wherein the tube is a cylinder-like element with a substantially circular cross section and the tube material is flexible and has a water vapour transmission rate below 0.50 g/m²/mm/24h as measured with the WVTR method as defined in the description.

2. The catheter package according to claim 1, wherein the tube material has a water vapour transmission rate below 0.30 g/m²/mm/24h as measured with the WVTR method as defined in the description.

3. The catheter package according to any of the preceding claims, wherein the composition of the tube material comprises a thermoplastic polymer.

4. The catheter package according to any of the preceding claims, wherein the composition of the tube material comprises a thermoplastic elastomer.

5. The catheter package according to claim 4, wherein the thermoplastic elastomer comprises a styrene block copolymer such as polystyrene-*block*-poly(ethylene/butylene)-*block*-polystyrene, polystyrene-*block*-poly(ethylene/propylene)-*block*-polystyrene, polystyrene-*block*-polybutadiene-*block*-polystyrene or polystyrene-*block*-polyisobutylene-*block*-polystyrene.

6. The catheter package according to claim 5, wherein the thermoplastic elastomer further comprises polypropylene and/or polyethylene, and optionally a plasticizer such as paraffin oil in a compound.

7. The catheter package according to any of the preceding claims, wherein the composition of the tube material comprises polybutene, polyisobutylene, cured or uncured butyl rubber, or block copolymer with polystyrene as the end blocks such as styrene-isobutylene-styrene.

8. The catheter package according to any of the preceding claims, wherein the composition of the tube material comprises polyethylene with a density of 0.900-0.920 g/cm³.

9. The catheter package according to claim 8, wherein the polyethylene is a low density polyethylene or a linear low density polyethylene.

10. The catheter package according to any of the preceding claims, wherein the tube is corrugated.

11. The catheter package according to any of the claims 1-9, wherein the tube is straight.

12. The catheter package according to any of the preceding claims, wherein the tube is a mono layer construction.

13. A catheter package set comprising
- a tube package,
- an intermittent catheter
- a swelling medium,
- and optionally a urine collection bag,
wherein the tube is a cylinder-like element with a substantially circular cross section and tube material is flexible and has a water vapour transmission rate below 0.50 g/m²/mm/24h as measured with the WVTR method as defined in the description.

14. The catheter package set according to claim 13, wherein the swelling medium is disposed within the tube package.

15. The catheter package set according to any of the claims 13-14, wherein the tube package is according to any of the claims 1 to 12.

16. The catheter package set according to any of the claims 13-15, wherein the intermittent catheter comprises a hydrophilic coating.

17. The catheter package set according to any of the claims 13-16, wherein the package set has been sterilized using irradiation while in contact with the swelling medium.

18. The catheter package set according to claim 17, wherein the set is sterilized using β-or y-irradiation.

## Patentansprüche

1. Katheterverpackung, gebildet als Schlauch für einen intermittierenden Katheter, wobei der Schlauch ein zylinderartiges Element mit einem im Wesentlichen kreisförmigen Querschnitt ist und das Schlauchmaterial flexibel ist und eine Wasserdampftransmissionsrate von unter 0,50 g/m²/mm/24 h, wie gemessen durch das wie in der Beschreibung definierte WVTR-Verfahren, aufweist.

2. Katheterverpackung gemäß Anspruch 1, wobei das Schlauchmaterial eine Wasserdampftransmissionsrate von unter 0,30 g/m²/mm/24 h, wie gemessen durch das wie in der Beschreibung definierte WVTR-Verfahren, aufweist.

3. Katheterverpackung gemäß einem der vorstehenden Ansprüche, wobei die Zusammensetzung des Schlauchmaterials ein thermoplastisches Polymer umfasst.

4. Katheterverpackung gemäß einem der vorstehenden Ansprüche, wobei die Zusammensetzung des Schlauchmaterials ein thermoplastisches Elastomer umfasst.

5. Katheterverpackung gemäß Anspruch 4, wobei das thermoplastische Elastomer ein Styrol-Blockcopolymer umfasst, wie z. B. Polystyrol*block*-poly(ethylen/butylen)-*block*-polystyrol, Polystyrol-*block*-poly(ethylen/propylen)-*block-*polystyrol, Polystyrol-*block*-polybutadien-*block-*polystyrol oder Polystyrol-*block*-polyisobutylen-*block*-polystyrol.

6. Katheterverpackung gemäß Anspruch 5, wobei das thermoplastische Elastomer ferner Polypropylen und/oder Polyethylen und gegebenenfalls einen Weichmacher, wie z. B. Paraffinöl in einer Verbindung, umfasst.

7. Katheterverpackung gemäß einem der vorstehenden Ansprüche, wobei die Zusammensetzung des Schlauchmaterials Polybuten, Polyisobutylen, gehärteten oder nichtgehärteten Butylgummi, oder Blockcopolymer mit Polystyrol als Endblöcke, wie z. B. Styrol-Isobutylen-Styrol, umfasst.

8. Katheterverpackung gemäß einem der vorstehenden Ansprüche, wobei die Zusammensetzung des Schlauchmaterials Polyethylen mit einer Dichte von 0, 900-0, 920 g/cm³ umfasst.

9. Katheterverpackung gemäß Anspruch 8, wobei das Polyethylen ein Polyethylen mit niedriger Dichte oder ein lineares Polyethylen mit niedriger Dichte ist.

10. Katheterverpackung gemäß einem der vorstehenden Ansprüche, wobei der Schlauch gewellt ist.

11. Katheterverpackung gemäß einem der Ansprüche 1-9, wobei der Schlauch gerade ist.

12. Katheterverpackung gemäß einem der vorstehenden Ansprüche, wobei der Schlauch einen einschichtigen Aufbau aufweist.

13. Katheterverpackungszusammenstellung umfassend
- eine Schlauchverpackung,
- einen intermittierenden Katheter,
- ein quellendes Medium,
- und gegebenenfalls einen Urinsammelbeutel,
wobei der Schlauch ein zylinderartiges Element mit einem im Wesentlichen kreisförmigen Querschnitt ist und das Schlauchmaterial flexibel ist und eine Wasserdampftransmissionsrate von unter 0,50 g/m²/mm/24 h, wie gemessen durch das wie in der Beschreibung definierte WVTR-Verfahren, aufweist.

14. Katheterverpackungszusammenstellung gemäß Anspruch 13, wobei das quellende Medium innerhalb der Schlauchverpackung angeordnet ist.

15. Katheterverpackungszusammenstellung gemäß einem der Ansprüche 13-14, wobei die Schlauchverpackung gemäß einem der Ansprüche 1 bis 12 ist.

16. Katheterverpackungszusammenstellung gemäß einem der Ansprüche 13-15, wobei der intermittierende Katheter einen hydrophilen Überzug umfasst.

17. Katheterverpackungszusammenstellung gemäß einem der Ansprüche 13-16, wobei die Verpackungszusammenstellung in Kontakt mit dem quellenden Medium unter Verwendung von Bestrahlung sterilisiert worden ist.

18. Katheterverpackungszusammenstellung gemäß Anspruch 17, wobei die Zusammenstellung unter Verwendung von β- oder γ-Strahlung sterilisiert ist.

## Revendications

1. Emballage de cathéter formé comme un tube pour un cathéter intermittent, dans lequel le tube est un élément en forme de cylindre qui présente une section transversale sensiblement circulaire, et le matériau de tube est flexible et présente une vitesse de transmission de vapeur d'eau inférieure à 0,50 g/m²/mm/24 heures mesurée en utilisant le procédé WVTR tel qu'il est défini dans la description.

2. Emballage de cathéter selon la revendication 1, dans lequel le matériau de tube présente une vitesse de transmission de vapeur d'eau inférieure à 0,30 g/m²/mm/24 heures mesurée en utilisant le procédé WVTR tel qu'il est défini dans la description.

3. Emballage de cathéter selon l'une quelconque des revendications précédentes, dans lequel la composition du matériau de tube comprend un polymère thermoplastique.

4. Emballage de cathéter selon l'une quelconque des revendications précédentes, dans lequel la composition du matériau de tube comprend un élastomère thermoplastique.

5. Emballage de cathéter selon la revendication 4, dans lequel l'élastomère thermoplastique comprend un copolymère bloc de styrène tel qu'un polystyrène-bloc-poly(éthylène/butylène)-bloc-polystyrène, un polystyrène-bloc-poly(éthylène/propylène)-bloc-polystyrène, un polystyrène-bloc-polybutadiène-bloc-polystyrène ou un polystyrène-bloc-polyisobutylène-bloc-polystyrène.

6. Emballage de cathéter selon la revendication 5, dans lequel l'élastomère thermoplastique comprend en outre du polypropylène et/ou du polyéthylène, et optionnellement un agent plastifiant tel que l'huile de paraffine dans un composé.

7. Emballage de cathéter selon l'une quelconque des revendications précédentes, dans lequel la composition du matériau de tube comprend du polybutène, du polyisobutylène, du caoutchouc butyle durci ou non durci, ou un copolymère bloc avec du polystyrène comme blocs d'extrémité, tels que le styrène-isobutylène-styrène.

8. Emballage de cathéter selon l'une quelconque des revendications précédentes, dans lequel la composition du matériau de tube comprend du polyéthylène avec une densité de 0,900 g/cm³ à 0,920 g/cm³.

9. Emballage de cathéter selon la revendication 8, dans lequel le polyéthylène est un polyéthylène à faible densité ou un polyéthylène linéaire à faible densité.

10. Emballage de cathéter selon l'une quelconque des revendications précédentes, dans lequel le tube est ondulé.

11. Emballage de cathéter selon l'une quelconque des revendications 1 à 9, dans lequel le tube est droit.

12. Emballage de cathéter selon l'une quelconque des revendications précédentes, dans lequel le tube est une structure monocouche.

13. Ensemble d'emballage de cathéter, comprenant:
- un emballage de cathéter,
- un cathéter intermittent,
- un agent de gonflement,
- et optionnellement une poche de collecte d'urine,
dans lequel le tube est un élément en forme de cylindre qui présente une section transversale sensiblement circulaire, et le matériau de tube est flexible et présente une vitesse de transmission de vapeur d'eau inférieure à 0,50 g/m²/mm/24 heures mesurée en utilisant le procédé WVTR tel qu'il est défini dans la description.

14. Ensemble d'emballage de cathéter selon la revendication 13, dans lequel l'agent de gonflement est disposé à l'intérieur de l'emballage de tube.

15. Ensemble d'emballage de cathéter selon l'une quelconque des revendications 13 ou 14, dans lequel l'emballage de tube est du type selon l'une quelconque des revendications 1 à 12.

16. Ensemble d'emballage de cathéter selon l'une quelconque des revendications 13 à 15, dans lequel le cathéter intermittent comprend un revêtement hydrophile.

17. Ensemble d'emballage de cathéter selon l'une quelconque des revendications 13 à 16, dans lequel l'ensemble d'emballage a été stérilisé en utilisant un rayonnement pendant qu'il était en contact avec l'agent de gonflement.

18. Ensemble d'emballage de cathéter selon la revendication 17, dans lequel l'ensemble est stérilisé en utilisant un rayonnement β ou un rayonnement γ.
